# EUROPÄISCHE PATENTSCHRIFT

(11) **EP 2 708 214 B1**
(45) Veröffentlichungstag und Bekanntmachung des Hinweises auf die Patenterteilung: **29.11.2017**
(21) Anmeldenummer: 13401101.4
(22) Anmeldetag: 09.09.2013
(51) Int. Cl.: A61F 13/00, A61F 13/02

(54) **Vorrichtung zur Durchführung einer therapeutischen und/oder kosmetischen Behandlung von Warzen oder dergleichen erhabenen, sich von der Hautoberfläche eines Lebewesens erhebenden Gewebeabnormalität**
Device for carrying out therapeutic and/or cosmetic treatment of warts or similar tissue abnormalities raised from the surface of the skin of a living being
Dispositif permettant de réaliser un traitement thérapeutique et/ou cosmétique de verrues ou autres anormalités tissulaires proéminentes existant à la surface de la peau d'un être vivant

(30) Priorität: 07.09.2012 DE 102012108375
(43) Veröffentlichungstag der Anmeldung: 19.03.2014
(73) Patentinhaber: Zalzadeh, Ario, 34516 Vöhl Thalitter (DE)
(72) Erfinder: Zalzadeh, Ario, 34516 Vöhl Thalitter (DE)
(74) Vertreter: Sprenger, Gerrit Lars Eike

(56) Entgegenhaltungen:
- WO-A1-2011/092595
- JP-A- 2003 144 442
- US-A- 4 860 746

## Beschreibung

Die Erfindung betrifft eine Vorrichtung zur Durchführung einer therapeutischen und/oder kosmetischen Behandlung von Warzen oder dergleichen erhabenen, sich von der Hautoberfläche eines Lebewesens erhebenden Gewebeabnormalitäten.

Im Stand der Technik sind eine Vielzahl von Vorrichtungen und Verfahren bekannt, mit denen Warzen oder dergleichen erhabenen, sich von der Hautoberfläche eines Lebewesens erhebenden Gewebeabnormalitäten mit dem Ziel der Entfernung oder Rückbildung derselben therapiert werden.

Zu diesen bekannten Verfahren zählt beispielsweise auch die chirurgische Beseitigung, welche natürlich einen massiven invasiven Eingriff in die umgebenden Haut- und Gewebebereiche der Warze beziehungsweise der erhabenen, sich von der Hautoberfläche eines Lebewesens erhebenden Gewebeabnormalität bedeuten. Durch einen solchen chirurgischen Eingriff sind offene Wunden unvermeidbar, deren Heilung individuell körperbedingt schwierig verlaufen kann und oftmals auch mit einer - insbesondere für Menschen aus ästhetischen Gründen - nicht akzeptablen Narbenbildung verbunden ist, insbesondere dann, wenn die zu entfernende Warze beziehungsweise Gewebeabnormalität auf Hautpartien angesiedelt ist, welche im Alltag nicht von Kleidung bedeckt sind, wie beispielsweise im Gesicht oder an den Händen einer Person. Ferner ist durch die offene Wunde bei einem solchen chirurgischen Verfahren auch immer latent eine Infektionsgefahr vorhanden, da der Blutkreislauf während der chirurgischen Behandlung zur Umwelt hin offen ist und somit Keime, Bakterien und/oder Viren in den Körper des Patienten eindringen können. Insbesondere bei Warzen, die über eine Infektion des jeweiligen Hautbereichs mit bestimmten warzenauslösenden Viren entstehen, besteht die Gefahr, dass bei einem solchen chirurgischen Eingriff umliegende Hautpartien mit dem entsprechenden Virus infiziert werden und sich dort erneut Warzen ausbilden, nachdem die ursprüngliche Warze entfernt werden konnte.

Nach einem anderen Verfahren, wie es beispielsweise in der EP 0 281 212 B1 beschrieben ist, werden Warzen mittels eines Kühlmittels vereist. Solche Vereisungen haben allerdings den Nachteil, dass die Warze umgebende gesunde Hautpartien gegebenenfalls auch von der Vereisung betroffen sind und sich in unästhetischer Weise weiß verfärben können oder es zu Blasenbildung beziehungsweise Verbrennungen kommen kann. Unter Umständen können diese ursprünglich gesunden Hautpartien ebenfalls wie die Warze absterben. Zudem ist die Dosierung problematisch, sodass auch eine Narbenbildung, Nervenschädigung oder auch eine Gewebeschädigung nicht auszuschließen ist. Auch kann es in dem Bereich der Warze nach deren Absterben zu Pigmentstörungen der neu gebildeten Haut kommen.

Ferner ist es auch noch bekannt, auf Warzen Tinkturen aufzutragen, was zu einer Rückbildung und schließlich zu einem Verschwinden der Warzen führen soll. Allerdings gehen damit lange Behandlungszeiten aufgrund dem notwendigen mehrmaligen wiederholten Auftragen von zum Teil mehreren Wochen einher. Bei häufigem Auftragen kann auch ein starkes Brennen besonders auf der die Warze umgebene gesunde Haut auftreten, wodurch auch mögliche Reizerscheinungen sowie Hautentzündungen entstehen können. Ferner ist auch hier die Dosierung problematisch, wobei Reste der erkrankten Hautstellen bestehen bleiben können und die Warze sich neu ausbilden kann.

Wie zuvor beschrieben alle diese Verfahren sind nicht nebenwirkungsfrei und hinterlassen z.T. Narben. Allerdings gibt es neben den oben erwähnten Verfahren von Alters her, vorwiegend im Hausgebrauch, auch eine Methode, bei der die Warze durch einen Bindfaden abgeschnürt wird, wodurch die Versorgung der Warze durch den Körper des Wirts unterbrochen wird und die Warze aufgrund von Unterversorgung abstirbt und, ohne eine Narben zu hinterlassen, abfällt. Allerdings ist die Handhabung eines solchen Bindfadens, der um die Warze gelegt werden muss, um diese ab zuschnüren, umständlich.

Ferner beschreibt auch die EP 1 833 382 B1 verschiedenste Vorrichtungen, mit welchen eine Unterversorgung einer Warze oder dergleichen erreicht wird, sodass auch mit einer solchen Vorrichtung eine Entfernung derselben ohne wesentliche Narbenbildung erreicht wird. Allerdings sind die dortigen Vorrichtungen zwar von relativ einfachem Aufbau, jedoch ist ihre Handhabung sehr benutzerunfreundlich, da die dortigen flächigen Gebilde je nach Ausführungsform unterschiedlich geknickt und/oder gefaltet werden müssen, um den erwünschten Behandlungserfolg zu erzielen.

Weiterhin zeigt die WO 2011/092595 A1 eine gattungsbildende Vorrichtung zur Durchführung einer therapeutischen Behandlung von Warzen oder dergleichen erhabenen, sich von der Hautoberfläche eines Lebewesens erhebenden Gewebeabnormalitäten. Diese Vorrichtung weist zwar auch ein flächiges Gebilde mit einer Oberseite und einer Unterseite auf. Allerdings bildet das dortige flächige Gebilde einen umlaufenden Rand an dem zum einen Enden von eine Öffnung für die Warze oder dergleichen bildende Randbereichen angeordnet sind. Zum Anderen sind zwischen dem umlaufenden Rand und den Randbereichen der Öffnung Stege angebracht, mit welchen nach einer Verformung des Gebildes und einer Aufnahme der Warze in der Öffnung eine Kraft auf die Randbereiche der Öffnung ausgeübt werden soll, sodass die Versorgung der Warze unterbrochen ist oder zumindest eine derartige Unterversorgung der Warze auftritt, dass die Folge deren Absterben ist. Allerdings ist diese Vorrichtung in ihrer Herstellung sehr aufwendig, da die Randbereiche der Öffnung, die Stege und der Rand des flächigen Gebildes in ihren Dimensionen derart aufeinader abgestimmt sein müssen, dass eine wirkungsvolle Unterversorgung der Warze gewährleistet ist. Hierzu müssen die Dimensionen des Randes, der Randebereiche und der Stege für unterschiedliche Größen und unterschiedliche Materialien des flächigen Gebildes aufwendig berechnet und dimensioniert werden.

Es ist daher Aufgabe der Erfindung, eine Vorrichtung zur Verfügung zu stellen, mit der eine Unterversorgung der Warze beziehungsweise dergleichen erhabenen, sich von der Hautoberfläche eines Lebewesens erhebenden Gewebeabnormalität bei gleichzeitiger einfacher Handhabung und ohne eine Verletzung der Warze beziehungsweise dergleichen erhabenen, sich von der Hautoberfläche eines Lebewesens erhebenden Gewebeabnormalität unabhängig von der Größe und den Materialien des flächigen Gebildes erreichen lässt.

Gelöst wird diese Aufgabe durch eine Vorrichtung mit allen Merkmalen des Patentanspruchs 1. Vorteilhafte Ausgestaltungen der Erfindung finden sich in den abhängigen Patentansprüchen 2 bis 15.

Die erfindungsgemäße Vorrichtung zur Durchführung einer therapeutischen Behandlung von Warzen oder dergleichen erhabenen, sich von der Hautoberfläche eines Lebewesens erhebenden Gewebeabnormalitäten besteht im Wesentlichen aus einem flächigen, einstückigen und einlagigen Gebilde, welches eine Oberseite, eine Unterseite und eine durch Randbereiche gebildete Öffnung aufweist, wobei die Vorrichtung aus einer Folie aus flexiblem Kunststoff, natürlichem Kautschuk, einem Thermoplasten oder einem Elastomer besteht und die Öffnung als Schlitz ausgebildet ist und wobei das Gebilde außerhalb des Bereichs der Öffnung öffnungsfrei ausgebildet ist. Das flächige Gebilde ist dabei derart verformbar, dass eine Warze beziehungsweise eine sich von der Hautoberfläche eines Lebewesens erhebende Gewebeabnormalität in der Öffnung positionierbar ist, wobei das Gebilde flächig mit seiner Unterseite auf der die Warze beziehungsweise die Gewebeabnormalität umgebende Haut aufliegt und die Randbereiche der Öffnung eine Kraft insbesondere eine Rückstellkraft auf die Warze beziehungsweise die Gewebeabnormalität ausüben. Unter flächigen Gebilde soll im Sinne der Erfindung nicht nur ein flaches Gebilde mit parallel zueinander und einer ebenen Fläche angeordneter Ober- und Unterseite gemeint sein. Vielmehr müssen die das Gebilde begrenzende Ober-und Unterseite nicht parallel zu einer ebenen Fläche angeordnet sein, sondern können auch als im Wesentlichen parallel verlaufende Krümmungen oder auch nicht parallel verlaufend, konisch zulaufend ausgebildet sein.

Die zu therapierende beziehungsweise zu entfernende Warze oder dergleichen erhabenen, sich von der Hautoberfläche eines Lebewesens erhebenden Gewebeabnormalität wird durch die Öffnung des flächigen Gebildes der erfindungsgemäßen Vorrichtung geführt. Nachdem die Warze beziehungsweise Gewebeabnormalität in die Öffnung des flächigen Gebildes der Vorrichtung eingelegt ist, wird das flächige Gebilde mit seiner Unterseite flach auf die umliegenden Hautpartien gelegt. Dadurch üben die Randbereiche der Öffnung des flächigen Gebildes auf die die Warze beziehungsweise die Gewebeabnormalität versorgenden Kapillaren beziehungsweise Gefäße eine Kraft auf, sodass die Kapillaren beziehungsweise Gefäße zumindest zum Teil verschlossen werden, sodass es zu einer Unterversorgung der Warze beziehungsweise der Gewebeabnormalität kommt. In der Regel stirbt in Folge dieser Unterversorgung die Warze beziehungsweise die Gewebeabnormalität nun innerhalb von zwölf Stunden ab, ohne dabei Körpersignale abzugeben. Nach etwa drei Tagen hat sich die Warze beziehungsweise die Gewebeabnormalität in der Regel vollständig vom Körper abgelöst. Nach etwa zwei Wochen ist die Haut im Bereich der ehemaligen Warze beziehungsweise Gewebeabnormalität derart abgeheilt beziehungsweise regeneriert, dass die ehemalige Existenz der Warze beziehungsweise Gewebeabnormalität nicht oder kaum mehr zu erkennen ist und ein kosmetisch und ästhetisch gutes Gesamtbild der Haut ohne Narbenbildung entstanden ist. Die Hautoberfläche ist nunmehr vollkommen genesen. Aufgrund der einfachen Anwendungsweise, die pro Warze beziehungsweise Gewebeabnormalität nur ein einziges Mal durchgeführt werden muss, benötigt man keine Spezialisten, Ärzte oder dergleichen medizinisch geschultes Personal, sondern kann diese Therapie selber vornehmen. Auch ist keine komplizierte Falt- oder Knickanleitung zu beachten. Zudem ist die erfindungsgemäße Vorrichtung in einfacher Weise schnell und kostengünstig, beispielsweise mittels bekannter Stanzverfahren aus einer Vielzahl von Materialien herstellbar. Patienten, die gegen bestimmte Inhaltsstoffe der herkömmlichen Verfahren überempfindlich sind, können die erfindungsgemäße Vorrichtung genauso benutzen, wie Schwangere, Stillende sowie Diabetiker oder blutverdünnende Medikamente einnehmende Patienten, die in der Regel alle von der Spontananwendung der aus dem Stand der Technik bekannten chirurgischen Verfahren, Vereisungsverfahren und der Behandlung mit Tinkturen ausgeschlossen sind. Der kosmetisch und optisch nachteilige ästhetische Eindruck dieser Verfahren wird durch die erfindungsgemäße Vorrichtung vermieden. Bestehende Warzen beziehungsweise Gewebeabnormalitäten können mit der erfindungsgemäßen Vorrichtung in allen möglichen Formen in einer einfachen und für jeden anzuwendenden Weise so korrigiert werden, dass ein glattes einwandfreies Hautbild entsteht. Körperliche und medizinische Nebenwirkungen sind nicht zu erwarten und ein Stück positive Lebensqualität wird den Betroffenen zurückgegeben beziehungsweise ermöglicht. Durch die Ausbildung der Öffnung als Schlitz oder dergleichen ist gewährleistet, dass die Randbereiche der Öffnung, nachdem die Warze beziehungsweise Gewebeabnormalität durch die Öffnung geführt und das flächige Gebilde flach auf den die Warze beziehungsweise Gewebeabnormalität umgebenden gesunden Hautpartien des Patienten abgelegt wurde, eine maximale Kraft auf die die Warze beziehungsweise die Gewebeabnormalität versorgenden Kapillaren oder Gefäße ausüben können. Insofern ist dadurch sicher gestellt, dass die gewünschte Unterversorgung der Warze beziehungsweise Gewebeabnormalität und somit deren Absterben eintritt. Abhängig von der Größe der zu therapierenden Warze beziehungsweise Gewebeabnormalität kann es natürlich auch vorgesehen sein, die Öffnung in einer anderen geometrischen Form, beispielsweise als Ellipse oder auch mit bogenförmigen, insbesondere kreisbogenförmigen Randbereichen auszubilden. Auch bei solchen Ausgestaltungen ist es möglich die gewünschte Unterversorgung, die zum Absterben der Warze beziehungsweise Gewebeabnormalität führt, zu erzielen. Von besonderem Vorteil ist dabei, wenn das als Folie ausgebildete Gebilde aus einem flexiblen Kunststoff oder natürlichem Kautschuk beziehungsweise aus einem Thermoplast oder einem Elastomer gebildet ist. Solche Materialien sind in vielfältiger Form vorhanden und für den erfindungsgemäßen Verwendungszweck einfach zu handhaben. Insbesondere lassen sich die flächigen Gebilde der erfindungsgemäßen Vorrichtung in einfacher Art und Weise als Folien herstellen.

Nach einem ersten vorteilhaften Gedanken der Erfindung ist das als Folie ausgebildete flächige Gebilde im Wesentlichen kreisförmig, oval, ellipsenförmig oder mehreckig ausgebildet. Grundsätzlich ist die geometrische Form des flächigen Gebildes anhand der zu behandelnden beziehungsweise zu therapierenden Gewebeabnormalität oder Warze derart zu wählen, dass das flächige Gebilde eine Stabilität aufweist die es den Randbereichen der Öffnung des flächigen Gebildes ermöglicht, die die Warze beziehungsweise die Gewebeabnormalität versorgenden Kapillaren oder Gefäße zumindest derart zu verschließen, dass die gewünschte Unterversorgung auftritt. In der Praxis werden daher verschiedene Größen und Formen der erfindungsgemäßen Vorrichtung aus verschiedenen Materialien, die der Fachmann für gängige Größen und unter Berücksichtigung des Gewebes der Warze dergleichen erhabenen, sich von der Hautoberfläche eines Lebewesens erhebenden Gewebeabnormalitäten auswählt.

In die gleiche Richtung zielt die Ausgestaltung des als Folie ausgebildeten flächigen Gebildes mit einer Breite entlang einer Längsachse des flächigen Gebildes, die mindestst zweimal so groß ist wie die des flächigen Gebildes. Auch hierdurch ist eine Anpassung der erfindungsgemäßen Vorrichtung hinsichtlich der Stabilität des flächigen Gebildes in Bezug auf Warzen und Gewebeabnormalitäten mit unterschiedlichen Größen und aus unterschiedlichen Gewebearten möglich.

Vorteilhafterweise ist das als Folie ausgebildete flächige Gebilde dabei im Wesentlichen entlang einer Längsachse derart verformbar, dass die als Schlitz oder dergleichen ausgebildete Öffnung eine geometrische Form annimmt, durch welche die Warze beziehungsweise die Gewebeabnormalität hindurchgeführt werden kann. Durch diese Maßnahme ist ein einfaches Einführen der Warze beziehungsweise Gewebeabnormalität in die Öffnung des flächigen Gebildes und somit in die erfindungsgemäße Vorrichtung erreicht, wodurch dessen Handhabung nochmals vereinfacht ist.

Beispielhaft sind nachfolgend einige Materialien aufgeführt, aus denen das als Folie ausgebildete flächige Gebilde hergestellt sein kann: ABS (Acrylnitril-Butadien-Styrol), APE (Aromatische Polyester), ASA(Acrylester-Styrol-Acrylnitril), BR(Butadien-Kautschuk), CA (Celluloseacetat), CN (Cellulosenitrat), COC (Cyclo-Olefin-Copolymere), CR (Chloropren-Kautschuk), CSM (chlorsulfoniertes Polyethylen), ECB (Ethylen-Copolymer-Bitumen), EP (Epoxidharz), EPDM (Äthylen-Propylen-Dien-Kautschuk), EPM (Ethylen-Propylen-Copolymer (Kautschuk)), ETFE (Ethylen-Tetrafluorethylen), EVA beziehungsweise EVM (Ethylenvinylacetat), FEP (Perfluor (Ethylen-Propylen-) Kunststoff), FKM beziehungsweise FPM (Fluor-Polymer-Kautschuk), FFKM beziehungsweise FFPM (Perfluorierter Kautschuk), FVMQ (Fluor-Silikon-Kautschuk), IIR (Butylkautschuk), IR (Isopren-Kautschuk), LCP (Liquid Crystal Polymer), MF (Melamin-Formaldehyd-Harz), MPF (Melamin-Phenol-Formaldehyd-Harz), NBR (Acrylnitril-Butadien-Kautschuk), NR (Naturkautschuk), PA (Polyamid), PAEK (Polyaryletherketon), PAI (Polyamidimid), PAN (Polyacrylnitril), PBT (Polybutylenterephthalat), PC (Polycarbonat), PCT (Polycyclohexylendimethylenterephthalat), PCTFE (Polychlortrifluorethylen), PE (Polyethylen), PEC (chloriertes Polyethylen), PE-HD)Polyethylen, hohe Dichte), PE-HMW (Polyethylen, hochmolekular, hohe Molmasse), PE-LD (Polyethylen, niedrige Dichte), PE-LLD (Polyethylen, linear, niedrige Dichte), PE-MD (Polyethylen, mittlere Dichte), PE-UHMW (Polyethylen, ultrahochmolekular, sehr hohe Molmasse), PEEK (Polyetheretherketon), PEI (Polyetherimid), PEK (Polyetherketon), PEN (Polyethylennaphthalat), PES (Polyethersulfon), PET (Polyethylenterephthalat), PF (Phenol-Formaldehyd-Harz), PFA (Perfluoralkoxylalkan), PFPE (Perfluorpolyether), PI (Polyimid), PIB (Polyisobuten), PMI (Polymethacrylimid), PMMA (Polymethylmethacrylat), PMP (Polymethylpenten), POM (Polyoxymethylen (Polyacetal)), PP (Polypropylen), PP-C (Polypropylen, Copolymer), PP-H(Polypropylen; Homopolymer), PPA (Polyphthalamid), PPE (Polyphenylenether), PPS (Polyphenylensulfid), PPY (Polypyrrol), PS (Polystyrol), PS-E (Polystyrol, expandierbar), PSU (Polysulfon), PTFE (Polytetrafluorethylen), PUR (Polyurethan), PVA (Polyvinylalkohol), PVAC (Polyvinylacetat), PVC (Polyvinylchlorid), PVDC (Polyvinylidenchlorid), PVDF (Polyvinylidenfluorid), PVF (Polyvinylfluorid), SAN (Styrol-Acrylnitril), SBR (Styrol-Butadien-Kautschuk), SI (Silikone), TPE (Thermoplastische Elastomere), UF (Harnstoff-Formaldehyd-Harz), UP (Ungesättigte Polyesterharze) oder VMQ (Silikon-Kautschuk). Obwohl das flächige Gebilde der erfindungsgemäßen Vorrichtung sowie die dazugehörigen Randbereiche der Öffnung nicht mit Körperflüssigkeiten in Kontakt kommen ist es sinnvoll das flächige Gebilde aus Polyethylen zu bilden, da dieser Kunststoff sich im medizintechnischen Bereich, insbesondere auch in der Infusionstherapie ohne wesentlich Nebenwirkungen etabliert hat, obwohl dort der Kontakt mit Körperflüssigkeiten gegeben ist. Ferner genügt Polyethylen allen in der Medizintechnik notwendigen Hygieneanforderungen und ist hautverträglich.

Als weiterhin vorteilhaft hat sich herausgestellt, dass die Randbereiche der Öffnung im Wesentlichen parallel zueinander angeordnet sind, wenn in der Öffnung keine Warze beziehungsweise eine sich von der Hautoberfläche eines Lebewesens erhebende Gewebeabnormalität positioniert ist. Eine solche Ausgestaltung der erfindungsgemäßen Vorrichtung gewährleistet, dass die Randbereiche genügende Kraft auf eine Warze beziehungsweise Gewebeabnormalität ausüben können, um deren Unterversorgung sicherzustellen, wenn diese in der Öffnung aufgenommen ist.

Allerdings ist es auch möglich diese Unterversorgung zu erzielen, wenn die Randbereiche der Öffnung im Wesentlichen bogenförmig, vorzugsweise kreisbogenförmig ausgebildet sind, wenn in der Öffnung keine Warze beziehungsweise keine sich von der Hautoberfläche eines Lebewesens erhebende Gewebeabnormalität positioniert ist. Diese Ausgestaltung bietet sich insbesondere bei größeren Warzen beziehungsweise Gewebeabnormalitäten an, da die Randbereiche der Öffnung sich dann besser an die Warze beziehungsweise die Gewebeanomalie andrücken können und somit die Unterversorgung derselben durch eine zumindest teilweises Verschließen der die Warze beziehungsweise die Gewebeanomalie versorgenden Kapillaren beziehungsweise Gefäßen sicherstellen.

Hierbei hat sich bewährt, dass sowohl im Fall der parallelen Ausbildung der Randbereiche der Öffnung als auch im Fall der im Wesentlichen bogenförmigen, vorzugsweise kreisbogenförmigen Ausbildung der Randbereiche der Öffnung, die Öffnung sich im Wesentlichen in der Mitte des flächigen Gebildes über etwa 40% bis 60% des Durchmessers beziehungsweise der Breite des flächigen Gebildes erstreckt.

Ein weiterer vorteilhafter Gedanke der Erfindung ist, dass die Randbereiche gegenüber dem als Folie ausgebildeten flächigen Gebilde verstärkt sind. Hierdurch ist gewährleistet, dass die zum zumindest teilweise Verschließen der die Warze beziehungsweise Gewebeabnormalität versorgenden Kapillare beziehungsweise Gefäße notwendige Kraft durch die Randbereiche aufgebracht werden kann, ohne dass diese zu viel nachgeben.

Je nach Größe und Struktur der Warze beziehungsweise Gewebeanomalie können die Randbereiche im Querschnitt eine rechteckige, kreisbogenförmige oder dreieckige Form aufweisen.

Nach einem weiteren Gedanken der Erfindung ist es vorgesehen, dass die erfindungsgemäße Vorrichtung Teil eines Sets ist, zu dem ein Fixierungsmittel zur Fixierung der Vorrichtung auf einem zu therapierenden Hautbereich eines Lebewesens gehört. Mit einem solchen Fixierungsmittel ist es in einfacher Weise möglich das flächige Gebilde der erfindungsgemäßen Vorrichtung in seinem Gebrauchszustand mit durch die Öffnung geführter Warze oder Gewebeanomalie flach auf der Haut des Patienten zu fixieren. Durch eine solche Fixierung ist sichergestellt, dass der Patient in seiner Bewegungsfreiheit nicht eingeschränkt ist und das flächige Gebilde der erfindungsgemäßen Vorrichtung trotzdem während der gesamten Therapie flächig auf der die Warze beziehungsweise Gewebeanomalie umgebenden Haut des Patienten aufliegt.

Vorteilhafterweise ist dabei das Fixierungsmittel als ein Pflaster ausgebildet ist. Hierdurch ist nicht nur das flächige Aufliegen des flächigen Gebildes auf der die Warze beziehungsweise Gewebeanomalie umgebenden Haut des Patienten während der gesamten Therapie sichergestellt. Vielmehr ist durch ein solches Pflaster auch die Warze beziehungsweise Gewebeanomalie sicher abgedeckt, sodass sie währen der Therapie nicht verletzt werden kann, was insbesondere bei Warzen zu einer Neuinfektion benachbarter Hautpartien führen könnte.

Nach einem weiteren Gedanken der Erfindung ist in dem Set zusätzlich ein Greifmittel zum Greifen einer Warze beziehungsweise einer sich von der Hautoberfläche eines Lebewesens erhebende Gewebeabnormalität enthalten ist, wobei das Greifmittel vorzugsweise als eine Pinzette ausgebildet ist.

Weitere Ziele, Vorteile, Merkmale und Anwendungsmöglichkeiten der vorliegenden Erfindung ergeben sich aus der nachfolgenden Beschreibung eines Ausführungsbeispiels anhand der Zeichnung. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1:: ein Ausführungsbeispiel einer erfindungsgemäßen Vorrichtung in einer Draufsicht,
- Figur 2:: das Ausführungsbeispiel der Figur 1 in einer Querschnittdarstellung,
- Figur 3:: das Ausführungsbeispiel der Figur 1 mit aufgespreizter Öffnung in einer Draufsicht und
- Figur 4:: das Ausführungsbeispiel gemäß Figur 3 in einer Querschnittdarstellung,

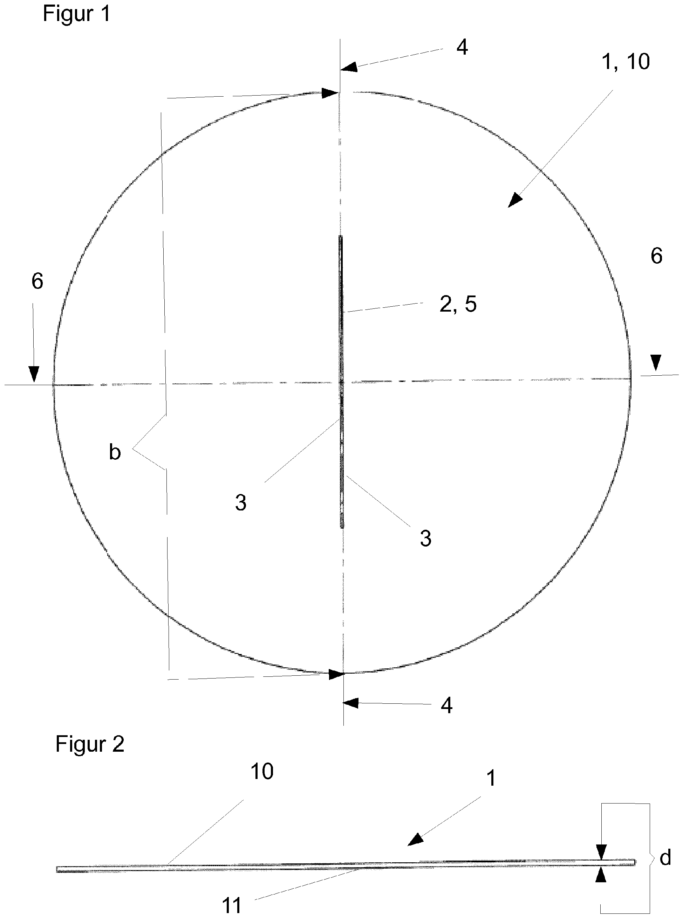

In den Figuren ist ein Ausführungsbeispiel einer erfindungsgemäßen Vorrichtung in verschiedenen Darstellungen gezeigt, wobei Figur 1 eine Draufsicht auf dieses Ausführungsbeispiel darstellt.

Im Wesentlichen besteht dieses Ausführungsbeispiel aus einem flächigen Gebilde1, welches vorliegend als kreisrunde scheibenförmige Folie mit einer Oberseite 10 und einer Unterseite 11 ausgebildet ist. Als Material für das flächige Gebilde 1 ist eine flexibler Kunststoff, vorzugsweise Polyethylen vorgesehen. Innerhalb des als kreisrunde scheibenförmige Folie ausgebildeten flächigen Gebildes 1 ist entlang einer Längsachse 4 eine Öffnung 2 in Form eines Schlitzes 5 eingebracht. Der Schlitz 5 erstreckt sich von der Mitte des als kreisrunde scheibenförmige Folie ausgebildeten flächigen Gebildes 1 über etwa 50% dessen Durchmessers. Die Öffnung 2 ist durch Randbereiche 3 begrenzt.

In Figur 2 ist das Ausführungsbeispiel der Figur 1 in einer Querschnittdarstellung gezeigt. Deutlich zu erkennen ist hierbei die flächige Ausgestaltung des Gebildes 1, dessen Durchmesser ein Vielfaches seiner Dicke beträgt.

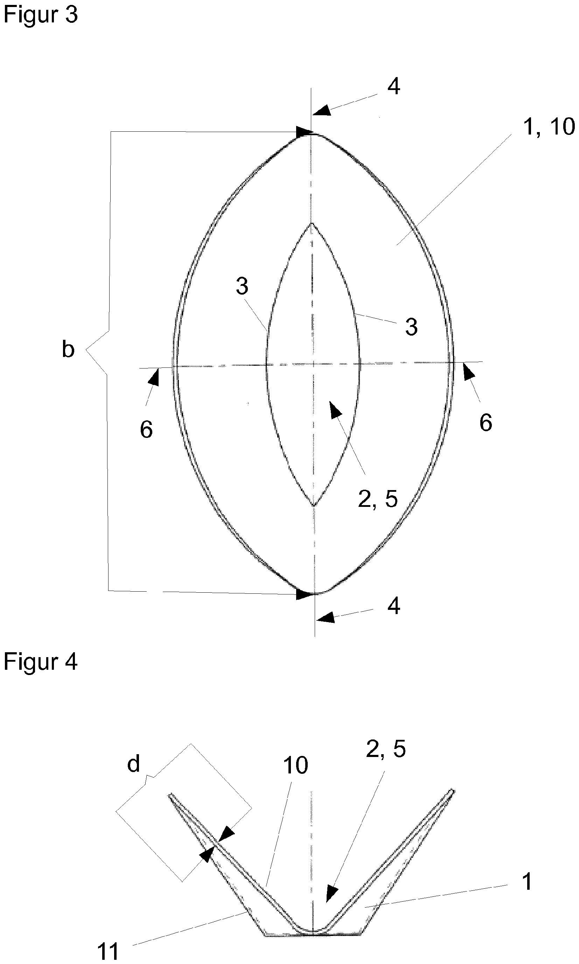

Die Figur 3 zeigt das Ausführungsbeispiel der Figur 1 im vorbereiteten Zustand zur Aufnahme einer Warze beziehungsweise einer Gewebeanomalie mit aufgeweiteter Öffnung 2, während Figur 4 eine Querschnittdarstellung dieses vorbereiteten Zustands dieses Ausführungsbeispiels darstellt.

Die Erfindung macht sich hierbei die Eigenschaft von Materialien zunutze, dass auf Biegung beanspruchte Körper deutlich nachgiebiger sind und damit elastischer, als direkt auf Zug und Druck beanspruchte Körper.

Im Folgenden wird nun beschrieben, wie das hier gezeigte Ausführungsbeispiel zur Therapiezwecken appliziert wird.

Durch Biegen des flächigen Gebildes 1 entlang einer sich über den Schlitz 5 erstreckenden Längsachse 4 um ca. 90° und gleichzeitiges Aufspreizen des Schlitzes 5 beziehungsweise der Öffnung 2 entlang einer Querachse 6 der Schlitz 5 beziehungsweise die Öffnung 2 aus der in den Figuren 1 und 2 dargestellten Form in ein fast kreisrundes Loch überführt, wie es in der Figur 3 dargestellt ist, durch das die abzutrennende Warze mit einer handelsüblichen Pinzette durch das Loch gezogen werden kann. Das Biegen des flächigen Gebildes 1 erfordert keinen großen Kraftaufwand, da die Restquerschnitte neben dem Schlitz 2 nur auf Biegung beansprucht werden und somit der Verformung nur einen kleinen Widerstand entgegensetzen.

Entsprechend der Biegebelastung entstehen in den Randbereichen 3 Zug- und Druckspannungen, während in den neutralen andern Bereichen des flächigen Gebildes 1 im Wesentlichen keine Spannungen auftreten. Durch die geringe Dicke beziehungsweise Höhe des flächigen Gebildes 1 sind die notwendigen Biegekräfte gering, sodass man das flächige Gebilde 1 leicht mit den Fingern aufbiegen kann.

Durch ein nun nachfolgendes Strecken des flächigen Gebildes 1, bei dem dieses mit seiner Unterseite auf der Hautoberfläche des Patienten um die zu behandelnde Warze abgelegt wird, versucht die Öffnung 2 nun wieder die Form eines Spalts 5 wie in Figur 1 dargestellt anzunehmen. Da aber nun in der Öffnung 2 die Warze beziehungsweise die Gewebeanomalie angeordnet ist, kann die Form des Schlitzes 2 nicht mehr vollständig eingenommen werden. Aufgrund der reinen Zugbelastung des flächigen Gebildes 1 entstehen daher nun extrem hohe Druckkräfte, welche durch die Randbereiche 3 der Öffnung 2 auf die Warze übertragen werden. Hierdurch werden zur Versorgung der Warze beziehungsweise der Gewebeanomalie dienende Kapillare beziehungsweise Gefäße im Gewebe des Patienten zumindest zum Teil verschlossen, sodass eine Unterversorgung der Warze beziehungsweise der Gewebeanomalie eintritt. Im Idealfall wird die Versorgung der Warze beziehungsweise Gewebeanomalie nahezu vollständig und wirkungsvoll unterbrochen.

Der komplette Restquerschnitt des flächigen Gebildes 1 ist nun im Bereich der Längsachse 4 mit einer Zugkraft beaufschlagt, sodass die Randbereiche 3 der Öffnung 2 eine Druckkraft auf die Warze beziehungsweise Gewebeanomalie ausübt. Auch dieser Streckvorgang kann mit vergleichsweise geringen Kräften durch einfaches Andrücken der aufgeklappten Bereiche des flächigen Gebildes 1 auf die Haut des Patienten mit den Fingern durchgeführt werden. Das flächige Gebilde 1 geht nunmehr in Selbsthemmung und verbleibt eigenständig in der gestreckten Position bis es später abgenommen wird.

Über die Materialeigenschaften und die Schlitzbreite können unterschiedliche Abpressdrücke der Randbereiche 3 erzeugt werden. Durch diese erfindungsgemäße Vorrichtung lassen sich Warzen, Gewebeanomalien beziehungsweise Gewebeabnormalitäten wirkungsvoll in Sekundenschnelle einfach in der Handhabung und wirkungsvoll einschnüren, sodass sie nach kurzer Zeit - in der Regel etwa 12 bis 24 Stunden - absterben und nachfolgend abfallen. Durch die schonende Behandlung regeneriert sich die Haut narbenfrei.

In den Figuren nicht dargestellt sind die weiteren Teile des erfindungsgemäßen Sets, bestehend aus einem als Pinzette ausgebildeten Greifmittels und einem als Pflaster ausgebildeten Fixierungsmittel. Das zuvor beschriebene Aufweiten der als Schlitz 5 ausgebildeten Öffnung gemäß Figur 1 hin zu der Form gemäß der Figur 3, kann dadurch erreicht werden, dass die Pinzette durch die Öffnung 2 des flächigen Gebildes 1 hindurch geführt wird und ihre Greifarme geöffnet werden. In dieser Position der Pinzette nimmt das flächige Gebilde 1 die in den Figuren 3 und 4 dargestellt Form an. Mit der Pinzette kann nunmehr die zu therapierende Warze gegriffen und das flächige Gebilde über die Warze geführt werden. Mit den Fingern kann nunmehr der Anwender das flächige Gebilde 1 wieder in die Position gemäß den Figuren 1 und 2 überführen, wo es in Selbsthemmung übergeht und die Randbereiche 3 der Öffnung 2 die Versorgung der Warze unterbinden.

Obwohl das flächige Gebilde nunmehr in Selbsthemmung übergegangen ist, bietet es sich an, das flächige Gebilde 1 mittels eines Pflasters als Fixierungsmittel auf der Haut des Patienten zu fixieren. Zum einen wird dadurch die Selbsthemmung des flächigen Gebildes 1 nochmals unterstützt. Zum anderen wird dadurch allerdings auch die in der Öffnung 2 fixierte Warze durch das Pflaster abgedeckt und vor äußerem Einflüßen wie beispielsweise Reibung, Verletzungen oder dergleichen geschützt.

### Bezugszeichenliste

- 1: flächiges Gebilde
- 2: Öffnung
- 3: Randbereich
- 4: Längsachse
- 5: Schlitz
- 6: Querachse
- 10: Oberseite
- 11: Unterseite
- d: Dicke des flächigen Gebildes 1
- b: Breite des flächigen Gebildes 1 entlang der Längsachse 4

## Patentansprüche

1. Vorrichtung zur Durchführung einer therapeutischen und/oder kosmetischen Behandlung von Warzen oder erhabenen, sich von der Hautoberfläche eines Lebewesens erhebenden Gewebeabnormalitäten mit einem flächigen, einstückigen und einlagigen Gebilde (1), welches eine Oberseite (10), eine Unterseite (11) und eine durch Randbereiche (3) gebildete Öffnung (2) aufweist, **dadurch gekennzeichnet, dass** die Vorrichtung aus einer Folie aus flexiblem Kunststoff, natürlichem Kautschuk, einem Thermoplasten oder einem Elastomer besteht und die Öffnung (2) als Schlitz ausgebildet ist, wobei das Gebilde außerhalb des Bereichs der Öffnung (2) öffnungsfrei ausgebildet ist.

2. Vorrichtung nach Anspruch 1, **dadurch gekennzeichnet, dass** das als Folie ausgebildete flächige Gebilde (1) im Wesentlichen kreisförmig, oval, ellipsenförmig oder mehreckig ausgebildet ist.

3. Vorrichtung nach einem der vorhergehenden Ansprüche, **dadurch gekennzeichnet, dass** die Dicke (d) des als Folie ausgebildeten flächigen Gebildes (1) und die Breite b des flächigen Gebildes (1) entlang einer Längsachse (4) folgende Relation aufweist: b ≥ 2 x d.

4. Vorrichtung nach einem der vorhergehenden Ansprüche, **dadurch gekennzeichnet, dass** das als Folie ausgebildete flächige Gebilde (1) im Wesentlichen entlang der Längsachse (4) derart verformbar ist, das die als Schlitz (5) oder dergleichen ausgebildete Öffnung (2) eine geometrische Form annimmt, durch welche die Warze beziehungsweise die Gewebeabnormalität hindurchgeführt werden kann.

5. Vorrichtung nach einem der vorhergehenden Ansprüche, **dadurch gekennzeichnet, dass** das als Folie ausgebildete Gebilde (1) aus ABS (Acrylnitril-Butadien-Styrol), APE (Aromatische Polyester), ASA(Acrylester-Styrol-Acrylnitril), BR(Butadien-Kautschuk), CA (Celluloseacetat), CN (Cellulosenitrat), COC (Cyclo-Olefin-Copolymere), CR (Chloropren-Kautschuk), CSM (chlorsulfoniertes Polyethylen), ECB (Ethylen-Copolymer-Bitumen), EP (Epoxidharz), EPDM (Äthylen-Propylen-Dien-Kautschuk), EPM (Ethylen-Propylen-Copolymer (Kautschuk)), ETFE (Ethylen-Tetrafluorethylen), EVA beziehungsweise EVM (Ethylenvinylacetat), FEP (Perfluor (Ethylen-Propylen-) Kunststoff), FKM beziehungsweise FPM (Fluor-Polymer-Kautschuk), FFKM beziehungsweise FFPM (Perfluorierter Kautschuk), FVMQ (Fluor-Silikon-Kautschuk), IIR (Butylkautschuk), IR (Isopren-Kautschuk), LCP (Liquid Crystal Polymer), MF (Melamin-Formaldehyd-Harz), MPF (Melamin-Phenol-Formaldehyd-Harz), NBR (Acrylnitril-Butadien-Kautschuk), NR (Naturkautschuk), PA (Polyamid), PAEK (Polyaryletherketon), PAI (Polyamidimid), PAN (Polyacrylnitril), PBT (Polybutylenterephthalat), PC (Polycarbonat), PCT (Polycyclohexylendimethylenterephthalat), PCTFE (Polychlortrifluorethylen), PE (Polyethylen), PEC (chloriertes Polyethylen), PE-HD)Polyethylen, hohe Dichte), PE-HMW (Polyethylen, hochmolekular, hohe Molmasse), PE-LD (Polyethylen, niedrige Dichte), PE-LLD (Polyethylen, linear, niedrige Dichte), PE-MD (Polyethylen, mittlere Dichte), PE-UHMW (Polyethylen, ultrahochmolekular, sehr hohe Molmasse), PEEK (Polyetheretherketon), PEI (Polyetherimid), PEK (Polyetherketon), PEN (Polyethylennaphthalat), PES (Polyethersulfon), PET (Polyethylenterephthalat), PF (Phenol-Formaldehyd-Harz), PFA (Perfluoralkoxylalkan), PFPE (Perfluorpolyether), PI (Polyimid), PIB (Polyisobuten), PMI (Polymethacrylimid), PMMA (Polymethylmethacrylat), PMP (Polymethylpenten), POM (Polyoxymethylen (Polyacetal)), PP (Polypropylen), PP-C (Polypropylen, Copolymer), PP-H(Polypropylen; Homopolymer), PPA (Polyphthalamid), PPE (Polyphenylenether), PPS (Polyphenylensulfid), PPY (Polypyrrol), PS (Polystyrol), PS-E (Polystyrol, expandierbar), PSU (Polysulfon), PTFE (Polytetrafluorethylen), PUR (Polyurethan), PVA (Polyvinylalkohol), PVAC (Polyvinylacetat), PVC (Polyvinylchlorid), PVDC (Polyvinylidenchlorid), PVDF (Polyvinylidenfluorid), PVF (Polyvinylfluorid), SAN (Styrol-Acrylnitril), SBR (Styrol-Butadien-Kautschuk), SI (Silikone), TPE (Thermoplastische Elastomere), UF (Harnstoff-Formaldehyd-Harz), UP (Ungesättigte Polyesterharze) oder VMQ (Silikon-Kautschuk) gebildet ist.

6. Vorrichtung nach einem der vorhergehenden Ansprüche, **dadurch gekennzeichnet, dass** die Randbereiche (3) der Öffnung (2) im Wesentlichen parallel zueinander angeordnet sind, wenn in der Öffnung (2) keine Warze beziehungsweise eine sich von der Hautoberfläche eines Lebewesens erhebende Gewebeabnormalität positioniert ist.

7. Vorrichtung nach einem der vorhergehenden Ansprüche, **dadurch gekennzeichnet, dass** die Randbereiche (3) der Öffnung (2) im Wesentlichen bogenförmig, vorzugsweise kreisbogenförmig ausgebildet sind, wenn in der Öffnung (2) keine Warze beziehungsweise keine sich von der Hautoberfläche eines Lebewesens erhebende Gewebeabnormalität positioniert ist.

8. Vorrichtung nach einem der vorhergehenden Ansprüche, **dadurch gekennzeichnet, dass** die Randbereiche (3) gegenüber dem als Folie ausgebildeten flächigen Gebilde (1) verstärkt ausgebildet sind.

9. Vorrichtung nach einem der vorhergehenden Ansprüche, **dadurch gekennzeichnet, dass** die Randbereiche (3) im Querschnitt eine rechteckige, kreisbogenförmige oder dreieckige Form aufweisen.

10. Set bestehend aus einer Vorrichtung nach einem der Ansprüche 1 bis 9 und einem Fixierungsmittel zur Fixierung dieser Vorrichtung auf einem zu therapierenden Hautbereich eines Lebewesens.

11. Set nach Anspruch 10, **dadurch gekennzeichnet, dass** Fixierungsmittel als ein Pflaster ausgebildet ist.

12. Set nach Anspruch 10 oder 11, **dadurch gekennzeichnet, dass** zusätzlich ein Greifmittel zum Greifen einer Warze beziehungsweise einer sich von der Hautoberfläche eines Lebewesens erhebende Gewebeabnormalität enthalten ist, wobei das Greifmittel vorzugsweise als eine Pinzette ausgebildet ist.

## Claims

1. Device for carrying out therapeutic and/or cosmetic treatment of warts, or raised tissue abnormalities rising above the skin surface of a living being, with a one-piece and single-ply sheetlike construct (1), which has an upper side (10), a lower side (11) and an opening (2) formed by edge regions (3), **characterized in that** the device consists of a self-supporting film of flexible plastic, natural rubber, a thermoplastic or an elastomer and the opening (2) is constructed as a slot, wherein the construct is constructed without an opening outside the region of opening (2).

2. Device according to Claim 1, **characterized in that** the sheetlike construct (1) constructed as a self-supporting film is constructed essentially circularly, ovally, in the shape of an ellipse or polyangularly.

3. Device according to either preceding claim, **characterized in that** the thickness (d) of the sheetlike construct (1) constructed as a self-supporting film and the width b of the sheetlike construct (1) along a longitudinal axis (4) satisfy the following relation: b ≥ 2 x d.

4. Device according to any preceding claim, **characterized in that** the sheetlike construct (1) constructed as a self-supporting film is essentially deformable along the longitudinal axis (4) such that the opening (2) constructed as a slot (5) or the like assumes a geometric shape wherethrough the wart or tissue abnormality can be guided.

5. Device according to any preceding claim, **characterized in that** the construct (1) constructed as a self-supporting film is formed of ABS (acrylonitrile-butadiene-styrene), APE (aromatic polyesters), ASA (acrylate-styrene-acrylonitrile), BR (butadiene rubber), CA (cellulose acetate), CN (cellulose nitrate), COC (cycloolefin copolymers), CR (chloroprene rubber), CSM (chlorosulfonated polyethylene), ECB (ethylene copolymer bitumen), EP (epoxy resin), EPDM (ethylene-propylene-diene monomer rubber), EPM (ethylene-propylene copolymer (rubber)), ETFE (ethylene-tetrafluoroethylene), EVA or EVM (ethylene-vinyl acetate), FEP (perfluoro(ethylene-propylene) plastic), FKM or FPM (fluoropolymer rubber), FFKM or FFPM (perfluorinated rubber), FVMQ (fluorosilicone rubber), IIR (butylrubber), IR (isoprene rubber), LCP (Liquid Crystal Polymer), MF (melamine-formaldehyde resin), MPF (melamine-phenol-formaldehyde resin), NBR (acrylonitrile-butadiene rubber), NR (natural rubber), PA (polyamide), PAEK (polyaryl ether ketone), PAI (polyamideimide), PAN (polyacrylonitrile), PBT (polybutylene terephthalate), PC (polycarbonate), PCT (polycyclohexylenedimethylene terephthalate), PCTFE (polychlorotrifluoroethylene), PE (polyethylene), PEC (chlorinated polyethylene), PE-HD (high density polyethylene), PE-HMW (high molecular weight polyethylene), PE-LD (low density polyethylene), PE-LLD (linear low density polyethylene), PE-MD (medium density polyethylene), PE-UHMW (ultra high molecular weight polyethylene), PEEK (polyether ether ketone), PEI (polyether imide), PEK (polyether ketone), PEN (polyethylene naphthalate), PES (polyether sulfone), PET (polyethylene terephthalate), PF (phenol-formaldehyde resin), PFA (perfluoroalkoxyalkane), PFPE (perfluoropolyether), PI (polyimide), PIB (polyisobutene), PMI (polymethyacrylimide), PMMA (polymethyl methacrylate), PMP (polymethyl pentene), POM (polyoxymethylene (polyacetal)), PP (polypropylene), PP-C (polypropylene copolymer), PP-H (polypropylene; homopolymer), PPA (polyphthalamide), PPE (polyphenylene ether), PPS (polyphenylene sulfide), PPY (polypyrrole), PS (polystyrene), PS-E (expandable polystyrene), PSU (polysulfone), PTFE (polytetrafluoroethylene), PUR (polyurethane), PVA (polyvinyl alcohol), PVAC (polyvinyl acetate), PVC (polyvinyl chloride), PVDC (polyvinylidene chloride), PVDF (polyvinylidene fluoride), PVF (polyvinyl fluoride), SAN (styrene-acrylonitrile), SBR (styrene-butadiene rubber), SI (silicones), TPE (thermoplastic elastomers), UF (urea-formaldehyde resin), UP (unsaturated polyester resins) or VMQ (silicone rubber).

6. Device according to any preceding claim, **characterized in that** the edge regions (3) of the opening (2) are essentially parallel to each other when no wart, or a tissue abnormality rising above the skin surface of a living being, is positioned in opening (2).

7. Device according to any preceding claim, **characterized in that** the edge regions (3) of the opening (2) are essentially shaped as an arc, preferably as a circular arc, when no wart, or a tissue abnormality rising above the skin surface of a living being, is positioned in opening (2).

8. Device according to any preceding claim, **characterized in that** the edge regions (3) are constructed to be thicker than the sheetlike construct (1) constructed as a self-supporting film.

9. Device according to any preceding claim, **characterized in that** the edge regions (3) have a rectangular, circularly arced or triangular shape in cross section.

10. Set consisting of a device according to any one of Claims 1 to 9 and a fixing means for fixing this device to a living being's skin region to be therapied.

11. Set according to Claim 10, **characterized in that** fixing means is constructed as a plaster.

12. Set according to Claim 10 or 11, **characterized in that** there is additionally included a gripping means for gripping a wart or a tissue abnormality rising above the skin surface of a living being, wherein the gripping means is preferably constructed as a pair of tweezers.

## Revendications

1. Dispositif pour la réalisation d'un traitement thérapeutique et/ou cosmétique de verrues ou d'anomalies tissulaires proéminentes existant à la surface de la peau d'un être vivant, ayant une structure (1) plate, d'un seul tenant et en une seule couche, qui comprend un côté supérieur (10), un côté inférieur (11) et une ouverture (2) formée par des zones de bord (3), **caractérisé en ce que** le dispositif est constitué par un film en plastique flexible, en caoutchouc naturel, en un thermoplastique ou en un élastomère, et l'ouverture (2) est configurée sous la forme d'une fente, la structure étant configurée sans ouverture en dehors de la zone de l'ouverture (2).

2. Dispositif selon la revendication 1, **caractérisé en ce que** la structure plate (1) configurée sous la forme d'un film est configurée essentiellement sous forme circulaire, ovale, ellipsoïdale ou polygonale.

3. Dispositif selon l'une quelconque des revendications précédentes, **caractérisé en ce que** l'épaisseur (d) de la structure plate (1) configurée sous la forme d'un film et la largeur b de la structure plate (1) le long d'un axe longitudinal (4) présentent la relation suivante : b ≥ 2 x d.

4. Dispositif selon l'une quelconque des revendications précédentes, **caractérisé en ce que** la structure plate (1) configurée sous la forme d'un film peut être déformée essentiellement le long de l'axe longitudinal (4) de sorte que l'ouverture (2) configurée sous la forme d'une fente (5) ou analogue prenne une forme géométrique au travers de laquelle la verrue ou l'anomalie tissulaire peut passer.

5. Dispositif selon l'une quelconque des revendications précédentes, **caractérisé en ce que** la structure (1) configurée sous la forme d'un film est formée par de l'ABS (acrylonitrile-butadiène-styrène), des APE (polyesters aromatiques), de l'ASA (ester acrylique-styrène-acrylonitrile), du BR (caoutchouc de butadiène), du CA (acétate de cellulose), du CN (nitrate de cellulose), des COC (copolymères de cyclo-oléfine), du CR (caoutchouc de chloroprène), du CSM (polyéthylène chlorosulfoné), des ECB (bitumes de copolymères d'éthylène), de l'EP (résine d'époxyde), de l'EPDM (caoutchouc d'éthylène-propylène-diène), de l'EPM (copolymère d'éthylène-propylène (caoutchouc)), de l'ETFE (éthylène-tétrafluoroéthylène), de l'EVA ou de l'EVM (éthylène-acétate de vinyle), du FEP (plastique perfluoro-(éthylène-propylène)), du FKM ou FPM (caoutchouc de polymère fluoré), du FFKM ou du FFPM (caoutchouc perfluoré), du FVMQ (caoutchouc de fluorosilicone), de l'IIR (caoutchouc de butyle), de l'IR (caoutchouc d'isoprène), du LCP (polymère cristallin liquide), de la MF (résine mélamine-formaldéhyde), de la MPF (résine mélamine-phénol-formaldéhyde), du NBR (caoutchouc d'acrylonitrile-butadiène), du NR (caoutchouc naturel), du PA (polyamide), de la PAEK (polyaryléther-cétone), du PAI (polyamide-imide), du PAN (polyacrylonitrile), du PBT (polybutylène-téréphtalate), du PC (polycarbonate), du PCT (polycyclohexylène-diméthylène-téréphtalate), du PCTFE (polychlorotrifluoroéthylène), du PE (polyéthylène), du PEC (polyéthylène chloré), du PE-HD (polyéthylène, haute densité), du PE-HMW (polyéthylène, masse moléculaire élevée), du PE-LD (polyéthylène, basse densité), du PE-LLD (polyéthylène, linéaire, basse densité), du PE-MD (polyéthylène, moyenne densité), du PE-UHMW (polyéthylène, masse moléculaire ultra-élevée), de la PEEK (polyéther-éther-cétone), du PEI (polyéther-imide), de la PEK (polyéther-cétone), du PEN (polyéthylène-naphtalate), de la PES (polyéther-sulfone), du PET (polyéthylène-téréphtalate), de la PF (résine phénol-formaldéhyde), du PFA (perfluoroalcoxylalcane), du PFPE (perfluoropolyéther), du PI (polyimide), du PIB (polyisobutène), du PMI (polyméthacrylimide), du PMMA (polyméthacrylate de méthyle), du PMP (polyméthylpentène), du POM (polyoxyméthylène (polyacétal)), du PP (polypropylène), du PP-C (polypropylène, copolymère), du PP-H (polypropylène ; homopolymère), du PPA (polyphtalamide), du PPE (polyéther de phénylène), du PPS (polysulfure de phénylène), du PPY (polypyrrol), du PS (polystyrène), du PS-E (polystyrène, expansible), de la PSU (polysulfone), du PTFE (polytétrafluoroéthylène), du PUR (polyuréthane), du PVA (alcool polyvinylique), du PVAC (polyacétate de vinyle), du PVC (polychlorure de vinyle), du PVDC (polychlorure de vinylidène), du PVDF (polyfluorure de vinylidène), du PVF (polyfluorure de vinyle), du SAN (styrène-acrylonitrile), du SBR (caoutchouc de styrène-butadiène), des SI (silicones), des TPE (élastomères thermoplastiques), de l'UF (résine urée-formaldéhyde), des UP (résines de polyester insaturées) ou du VMQ (caoutchouc de silicone).

6. Dispositif selon l'une quelconque des revendications précédentes, **caractérisé en ce que** les zones de bord (3) de l'ouverture (2) sont agencées essentiellement parallèlement les unes aux autres lorsqu'une verrue ou une anomalie tissulaire existant à la surface de la peau d'un être vivant n'est pas positionnée dans l'ouverture (2).

7. Dispositif selon l'une quelconque des revendications précédentes, **caractérisé en ce que** les zones de bord (3) de l'ouverture (2) sont configurées essentiellement sous la forme d'un arc, de préférence sous la forme d'un arc circulaire, lorsqu'une verrue ou une anomalie tissulaire existant à la surface de la peau d'un être vivant n'est pas positionnée dans l'ouverture (2).

8. Dispositif selon l'une quelconque des revendications précédentes, **caractérisé en ce que** les zones de bord (3) sont configurées sous forme renforcée par rapport à la structure plate (1) configurée sous la forme d'un film.

9. Dispositif selon l'une quelconque des revendications précédentes, **caractérisé en ce que** les zones de bord (3) présentent dans la section transversale une forme rectangulaire, en arc circulaire ou triangulaire.

10. Ensemble constitué par un dispositif selon l'une quelconque des revendications 1 à 9 et un moyen de fixation pour la fixation de ce dispositif sur une zone de peau à traiter d'un être vivant.

11. Ensemble selon la revendication 10, **caractérisé en ce que** le moyen de fixation est configuré sous la forme d'un pansement.

12. Ensemble selon la revendication 10 ou 11, **caractérisé en ce qu'**un moyen de préhension pour la préhension d'une verrue ou d'une anomalie tissulaire existant à la surface de la peau d'un être vivant est en outre contenu, le moyen de préhension étant de préférence configuré sous la forme d'une pincette.
